# EUROPEAN PATENT APPLICATION

(11) **EP 3 584 798 A1**
(43) Date of publication of application: **25.12.2019**
(21) Application number: 18178412.5
(22) Date of filing: 19.06.2018
(51) Int. Cl.: G16H 40/63, G16H 10/60

(54) **PREPARING TIME RELATED MEDICAL DEVICE DATA**

(71) Applicant: Tecpharma Licensing AG, 3401 Burgdorf (CH)
(72) Inventor: Köhli, Andreas, 2563 Ipsach (CH); Lindegger, Stefan, 4950 Huttwil (CH)

(57) **Abstract**

The invention relates to a method, preferable a computer-implementable method, for preparing, by a computing device (1), time related data from a medical device (3, 4) comprising the steps of computing a time difference between a synchronized time of the computing device and a time of the medical device connected to the computing device, wherein the synchronized time of the computing device is synchronized with a reference time provided by a time server (2); comparing the time difference with a predefined condition; importing or not data with a time stamp from the medical device depending on the comparison and generating, if imported, a converted time stamp from the time stamp of the imported data and based on the synchronized time of the computing device.

## Description

### FIELD OF THE INVENTION

The present invention relates to a computer implementable method, to a computer program and to a computing device for preparing time related data from a medical device connected to the computing device. The computing device imports data along with a time stamp from the medical device and converts the time stamp to a reference time.

### BACKGROUND OF THE INVENTION

Diabetes management systems known in the art usually comprise a medical device and a computing device such as a personal desktop computer, a mobile phone, a tablet or the like communicatively connected to the medical device. The medical device is, for example, a blood glucose measuring system, an infusion system, an injection system or an electronic module attachable to an infusion or injection system. The computing device may import, process, store and display data recorded by the medical devices and stored in a logbook in the medical device. Such medical device data may relate to events, for example, to a blood glucose measurement, an insulin delivery to the user or a change of the settings of the medical device. The medical device data comprises event data or information about the event itself as well as a time stamp stored along with the event data. The computing device analyses the medical device data related to the events and displays the events in graphs and tables over a time axis. For example, measured blood glucose values and the amount of a delivered drug are shown in a graph. Furthermore, a cumulated quantity of insulin delivered to the user, the so-called insulin on board, may be calculated based on the imported data including the time stamps. Other therapies such as hormone treatments likewise benefit from medical devices for subcutaneous administration of active ingredients, and produce event data being further evaluated in consolidated form by a computing device. In any case, the reliability of the data with the corresponding time stamps is essential for the further analysis of the data recorded in the medical device.

As long as all times and clocks in the medical devices and in the computing device are synchronized to a common time the processing of the imported data in the computing device works fine. However, problems with processing of the time related data may arise if a time of the medical device differs from a time in the importing computing device or if a time of a first medical device differs from a time of a second medical device. Furthermore, time changes of the time of a medical device may cause problems because in this case some of the time stamps may relate to the time prior to the time change while other time stamps may relate to the time after the time change. In both cases the data may not be correctly displayed as a function of time because the absolute time and the relative time for one event to another may be false or even the time stamps are not in the correct chronological order.

Methods for preparing time related data from different medical devices are known in the art. For example, WO 2016/087714 A1 discloses a method for monitoring of a glucose level of a user. A server communicates with blood glucose meters over internet. The blood glucose meters send their own current time along with measurement values and time stamps. The server then compares the current time sent by the glucose meter with a server time, detect a possible discrepancy and adjusts the timestamps to a known and correct reference time, such as the Universal Coordinated Time (UTC).

EP 2 988 672 B1 discloses a diabetes management system comprising a plurality of blood glucose meter in data communication with a diabetes management application residing on a mobile phone. Each meter has its internal real time clock (RTC) and the mobile phone has its internal phone time, which is set relative to GMT if the mobile phone is connected to a network. When the mobile phone is not connected to a network, the phone time is set to be equal to an RTC time of a meter. For each meter the diabetes management application determines a delta time between the phone time and the RTC of the meter. The management application stores received measurement values with RTC time stamps from the meter along with the corresponding computed time delta. Consequently, the management application computes a new time stamp for each measuring value by adding the time delta to the corresponding RTC time stamp. The measuring values are stored along with the computed new time stamp.

These prior art approaches focus on detecting a time difference, importing the data with the time stamps and converting the time stamp based on the time difference and based on a time of the importing system or a reference time. However, even with a conversion of the time stamps to a reference time a misalignment of time related data cannot be excluded because the time of a medical device or the time of the importing system may be wrong to an extent causing a time difference of one or more days, or the time stamps may be unusable because of an error or a time reset in the medical device or in the importing system or because of a manual time change in the medical device. Such extraordinary circumstances leading to ambiguous time stamps are not considered by the methods known in the art. Consequently, data with an incorrect time stamp may be processed by the computing device leading to incorrect results of the data analysis.

### SUMMARY OF THE INVENTION

It is an objective of the invention to enable processing of time related data in a correct temporal order, to improve data quality, and to prevent erroneous data analysis in circumstances in which the data originates from a device with a non-synchronized time.

These objectives are achieved by a method, a computer program product, and a computing device according to the respective independent claims. Preferred embodiments are evident from the dependent claims.

According to the invention a method, preferably a computer-implementable method, for preparing, processing, handling, or evaluating, by a computing device, time related data from a medical device connected to the computing device comprises the steps of
a. computing a time difference between a synchronized time of a computer program executed on the computing device and a time of the medical device, wherein the synchronized time of the computer program is synchronized with a reference time, preferably provided by a time server;
b. comparing the time difference with a predefined condition;
c. depending on the comparison, importing or not importing into the computing device, data with a time stamp from the medical device, and
d. generating, if imported, a converted time stamp from the time stamp of the imported data and based on the synchronized time of the computing device.

The invention relates further to a computer program product comprising instructions which, when executed by the computing device, cause the computing device to carry out the method as descripted above. Furthermore, the invention relates to a computing device comprising processing means configured or programed for carrying out the steps of the method as descripted above.

The computing device computes, and/or monitors, a time difference between an assumed synchronized time or clock of the computer program and a time or clock of the medical device by subtracting simultaneous time or clock readings. The computing device compares the computed time difference with a predefined condition or criteria, and based upon the result of the comparison, time related medical device data are imported and processed automatically without human intervention, or the importing of the data from the medical device is at least temporarily blocked by the computing device. In other words, the medical device data are validated and the data are imported selectively based on a criterion relating to the time difference. This ensures that only data are processed that meet the predefined condition, and data with time stamps from a medical device time or clock with ambiguous or questionable settings and/or otherwise relating to extraordinary circumstances is sorted out. As data are imported selectively the amount of data is reduced allowing a faster import as compared to a data validation after import.

The possibility of importing time related data based on a condition relating to a time difference is essential for subsequent processing and displaying the data by the computing device. The data may comprise, for example, information about a bodily fluid, such as blood glucose, or information about the amount, type and time of delivery of a drug to the user. In case such information are imported with an incorrect or ambiguous time stamp or with a time stamp being outside of a period of time of interest a subsequent computation based on such data may lead to a misleading or even wrong result. If the computed time difference is compared with a predefined condition and time related data are imported depending on the result of such a comparison a false or misleading computation of the data may be avoided.

The term "medical device" refers to a measuring device being capable of measuring bodily fluid, or to a drug delivery device for delivering, injecting, administering, infusing or dispensing substances from a product container into the skin of a patient. In insulin therapy, exemplary measuring devices include a blood glucose meter (BGM) for occasional measurement of the glucose level or a continuous glucose monitoring (CGM) system for continuously measuring the glucose level, both providing glucose readings as medical device data. A drug delivery device may be an infusion system or an injection system. For example, the drug delivery device may be a conventional medical drug pump such as an insulin pump with tubing or it may be a drug patch pump without tubing that is attachable directly onto the skin of the user, both providing basal rates and delivered bolus values as medical device data. The patch pump may communicate with a software application on an external device, which external device for the purpose of this invention may also be part of the medical device. Furthermore, the term "medical device" may refer to an electronic injection device adapted to record and to provide injection data or to an electronic module attachable to a delivery device. The electronic injection device may be an electronic injection pen like a reusable pen or it may be a patch injector applicable onto the skin of the user for the duration of the injection. The electronic module attachable to an injection device is also termed add-on, smart device, or smart module. The electronic module and the electronic injection device are adapted to recognize events and functions of the injection process and to provide medical device data such as a delivered dose amount, quality, time of injection, and circumstance of the injection process.

The medical device is usually not connected to a time server and thus unable to synchronize an internal time of the medical device with a reference time on its own motion without user input.

A computing device for executing the steps of the method may include any computing device capable of processing data such as a personal desktop computer, a portable laptop, a mobile phone or smart phone and a tablet computer or the like. Preferably, the computing device is adapted to send the processed or generated data to a display for showing the data to a user. The computer program is a software program running on the computing device. If the computing device is a mobile device the computer program is preferably a mobile software application adapted to be installed on the mobile device, e. g. on the smart phone or tablet.

In the present description some of the steps of the method according to the invention are descripted as executed by the computing device. However, it is to be understood that the computing device does not carry out the steps on its own motion but rather the executed computer program causes the computing device to carry out the mentioned steps. As mentioned above, the computer program is preferably residing and running on suitable storage and processing means of the computing device.

According to the invention the computer program maintains a synchronized time or clock which is synchronized with a reference time. The computer program and/or the computing device itself may independently provide and/or access other time-bases, counters, or clocks which in turn may or may not be synchronized with said synchronized time of the computer program.

A time base of the computing device or the medical device is, for example, a computer clock in the form of an integrated circuit that keeps track of time or a real time clock (RTC) of the device. Alternatively or additionally the time base may be a hardware clock or a so-called "system up time" provided by a counter of the device, wherein the counter counts the seconds since a last startup of the device.

The term "reference time" is to be understood as a time to which a plurality of clocks or times are synchronized to. The reference time may be a time standard like Greenwich Mean Time (GMT) or the Coordinated Universal Time (UTC) or a local reference time that is used as a reference time for a local area. The reference time is preferably provided by an external time server, which is physically separate to the computing device and to the medical device. Alternatively, the reference time may be calculated based on previously stored time data relating to the reference time.

The time stamp is an indication of time relating to an event occurring in the medical device. Preferably, the medical device records event data along with the time at which the event occurs, so that each event is stored in the medical device along with a corresponding time stamp. Preferably, the originally imported time stamp and the generated converted time stamp are stored by the computing device in a storage of the computing device. Thereby, the event is stored along with the two time stamps. Thus, both time stamps are available for any later computation. In a preferred embodiment both time stamps are stored in the chronological order in a storage of the computing device.

The computed time difference may be updated at any rate, preferably at least every 24 hours, or generally at the same rate as the re-synchronization of the synchronized time of the computer program takes place. Accordingly, the time difference may be computed for each event and corresponding event data individually, or be computed and applied collectively for a plurality of events occurring within a certain time range corresponding to the update rate.

The synchronized time of the computer program is synchronized or aligned with the reference time. The synchronized time does not have to be equal to the reference time but may involve a time base of the computer program with a well-defined relation to the reference time. For example, a constant time difference or a constant time delta between a time base of the computer program and the reference time may be computed and stored. In this case, the time base of the computer program qualifies as a synchronized time because it may be converted to the reference time by adding the computed and stored time delta.

Preferably, the synchronized time of the computer program is being re-synchronized periodically, preferably at least once within 24 hours. As the time base of the computer program may drift, periodical re-synchronization ensures that the time lag of the synchronized time of the computer program to the reference time does not become arbitrarily large. Therefore, the computed time difference essentially corresponds to the time difference between the reference time and the time of the medical device. Alternatively, the time of the computer program may be re-synchronized non-periodically, for example, it may be re-synchronized just at startup of the computing device.

The medical device may be connected to the computing device wirelessly, for example by a wireless local area network (WLAN) or by another short or near range wireless communication technology such as Bluetooth, ZigBee, WiMAX or NFC. Alternatively the medical device may be connected by wire, for example by a USB cable.

The predefined condition applied to, or evaluated with, the computed time difference may be, for example, a time threshold, a time range or a specific format for the time difference. The condition, for example, may be met if the computed time difference is greater or equal or just greater than the threshold or if the computed time difference is equal or smaller than the threshold. In another embodiment the condition may be met if the computed time difference is just greater or smaller than the threshold. If the condition is a time range the condition may be met if the time difference is within the range excluding the start and end value of that range.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The reference time is preferably the Coordinated Universal Time (UTC). The UTC is an international time standard which does not observe daylight saving time. UTC time server are easily accessible via internet. Therefore, UTC is well suited for a reference time. In an alternative embodiment the reference time may be a local or national legal time standard such as the Central European Summer Time (CEST).

In a preferred embodiment the condition includes a first threshold preferably equal to 24 hours, wherein the medical device data are not imported if an absolute value of the time difference exceeds the first threshold. That means the computing device does not import the data from the medical device having a time which differs as an absolute value by more than 24 hours from the time of the computing device. An absolute time difference equal or smaller than 24 hours can be caused by a time setting of a different time zone. For example, the Central European Time (CET) is 5 hours before Eastern Time (ET). However, a time difference exceeding 24 hours may not be simply due to different time zones and thus the time of the medical device and/or of the computing device is erroneous to the extent that manual or human intervention is unavoidable in order to reconcile the time of the medical device and the time of the computing device.

Alternatively, the condition may be a time interval and the data are only imported if the time difference is being within the time interval. In another embodiment the condition may be a time data format. In this case, for example, the data may not be imported if the computed time difference does not correspond or is not consistent with a specific data format. Such a non-consistent data format may be, for example, generated by the medical device if an error occurs during recording the event and the time stamps by the medical device.

Preferably, the condition includes a second threshold and the computing device informs the user if an absolute value of the time difference exceeds the second threshold. For example, the user is informed if the absolute value of the time difference exceeds a small value of a few seconds generally attributable to a time drift. Preferably, the computing device does not import any data if the absolute value of the time difference exceeds the predefined second threshold unless the user explicitly confirms acceptability of the time difference and manually validates the time stamp concerned. In a preferred embodiment such a second threshold is set to ten minutes. The computing device can inform the user, for example, by displaying a message on a display of the computing device.

After confirmation by the user the data are imported and a converted time stamp is calculated despite of the time difference exceeding the second threshold. The request for human confirmation is a precautionary measure allowing to override an automated condition-based non-import decision of the computing device.

Advantageously, the generation of the converted time stamp comprises the step of adding the time difference to the time stamp of the imported data. The time difference may be a positive or negative value. That means the term "adding" comprises an addition of a positive value to the time stamp as well as a subtraction of a value from the time stamp.

In a preferred embodiment the converted time stamp indicates the time instant expressed in reference time when the recorded event in the medical device took place. However, the conversion of the imported time stamp is not mandatorily limited to adding the time difference. The conversion may comprise additional computation steps such as transforming the imported data format to another data format.

Alternatively or additionally the imported time stamp may be converted to a further time base. For example, the converted time stamp may be additionally converted to a display time of the computing device or the computer program. This may be useful, for example, if the computing device comprises its own local or display time, which differs from the reference time. This display time may be set manually by the user in order to adjust the time of the computing device to a local time zone. Hence, the user may wish to display the imported, converted and processed data in the display time. This requires an additional converting step of the converted time stamp.

If a time server providing the reference time is not accessible or temporarily not available the reference time for synchronizing the time of the computer program is preferably computed by adding a time offset to a computer program system up time. The computer program system up time may be identical with or different from the time base of the computer program that serves as a basis for the synchronized time of the computer program. Preferably, the time offset is a previously computed and stored difference between the computer program system up time and the synchronized time of the computer program or between the former and, while available from the time server, the reference time. Thus, the reference time can be reconstructed without access to the time server. This may be necessary if, for example, the connection from the computer program to the internet is interrupted, if the time server is temporary not connected to the internet or if the time server is shut down.

Advantageously, in this context, the computer program verifies at startup of the computer program if the computer program system up time has been reset since the last instant at which the time offset has been computed. If the computer system up time has in fact been reset, for instance at a reboot of the computing device, the computer program up time may not be used by the computer program for reconstructing the reference time in case the time server is not accessible or not available. A reset of the computer system up time may be identified by comparing a current time of the computer program with a time stamp of the last startup of the computer program. The computer program system up time may be, for example, a time counter or a real time clock.

Alternatively, in this context, if the time server is not available and specifically if the time of the computing device cannot be used either, for example, if the computing device has been rebooted and thus the time has been reset, the reference time for synchronizing the time of the computer program is preferably computed by adding a time offset to a time base of the medical device. The time base of the medical device may be a local time of the medical device, which may be set manually by the user in order to adjust the local time of the medical device to a time zone. Alternatively, the time base of the medical device may be a system up time of the medical device, in which case a non-reset verification as previously described may be helpful. Preferably, the time offset is a previously computed and stored difference between the time base of the medical device and the reference time. The time offset may be stored at the medical device and provided to the computing device together with a current time of the medical device upon request, in view of a reconstruction of the reference time without access to the time server.

However, the two above mentioned reconstructions of the reference time should be exceptional. Under normal circumstances the time of the computer program can be synchronized with the reference time provided by the time server.

In a preferred embodiment, the imported medical device data are related to an event, preferably to a measurement event of a bodily fluid, a delivery event of a drug, or a time change event concerning a local time or clock setting of the medical device. The event occurs in the medical device and is preferably recorded and stored in a logbook of the medical device along with a time stamp relating to the event. Hence, the recorded and stored data preferably comprises the data relating to the event and a time stamp generated in a time base of the medical device. That allows to process the event data in a chronological order.

In a preferred embodiment the data comprises the event as a measured value of the blood glucose level of the user with a time stamp when the blood glucose measurement was carried out. Furthermore, the data may comprise the event as a delivered amount of drug and a time stamp indicating the time of the delivery of the drug. A manual time change event of a local time preferably comprises a time stamp before the time change (previous time) and an additional time stamp with the changed local time of the medical device.

If an event relating to a time change or correction of the local time of the medical device from a previous time to a new time is identified by the importing computing device, the computing device may retroactively change the time stamps of the events recorded prior to the time change event. A delta time as the difference between the new time and the previous time of the medical device may be added to the concerned time stamps to that purpose. This delta time may be a positive or a negative value resulting in either an addition of the delta time to the time stamp or resulting in a subtraction of the delta time from the time stamp. Due to the correction of the time stamp generated with the previous time (the time before the time change) all imported time stamps are in a correct order to each other irrespective of any time changes carried out in the medical device.

If the imported time stamp is not within a predefined expected or plausible range the time stamp or the event data related to this time stamp may be marked or flagged. A time stamp is not in an expected or plausible range, for example, if it is generated before a reset of the time of the medical device, if it has a number which does not fit in a chronological order to other imported time stamps, if the value of the time stamp is zero or if the time stamp comprises itself a marker indicating that the medical device was in error state when the event along with the time stamp was recorded by the medical device or indicating that the time stamp has been generated from different time sources.

The marking of time stamps by the computing device allows to process, store and/or display corresponding event data different from unmarked data. For example, marked time stamps or marked data can be processed with lower priority or marked time stamps are not displayed by default but only on request of the user.

In a preferred embodiment a time stamp being not within a predefined expected range is marked as invalid or the data related to that time stamp are marked as invalid by the computing device. Such marked data are not used for computation by the computer program in the computing device. If, for example, the medical device is an insulin pump and the marked data comprise information about the delivery of insulin, these data are not used for the computation of the insulin on board. This ensures that only actual data are used for processing in the computing device. In a preferred embodiment, the medical device is a blood glucose measuring device or an infusion pump. The infusion pump may be a conventional drug infusion pump with a tubing or a patch pump without tubing but attachable directly onto the skin of the user.

In an alternative embodiment the medical device may be an electronic injection pen, a patch injector or an electronic module, for example in the form of an add-on which is attachable to an injection device and adapted to record and store parameter settings, dose settings and injections carried out with the injection device.

Preferably, the computing device is a mobile device such as a mobile phone, a tablet computer, a smart watch, a laptop computer or the like. If the computing device is a mobile device the computer program is a mobile application running on the mobile device. In an alternative embodiment the computing device is a stationary personal desktop computer.

In this case the above mentioned computer program is a desktop computer software program.

### BRIEF DESCRIPTION OF THE DRAWINGS

The subject-matter of the invention will be explained in more detail in the following text with reference to preferred exemplary embodiments as illustrated in the attached drawings, of which
- Fig. 1: schematically depicts an arrangement with a mobile phone connected to a UTC time server, an insulin patch pump, and a blood glucose meter;
- Fig. 2: schematically depicts another embodiment with a desktop computer connected to a UTC time server, an insulin pump with tubing, and a blood glucose meter.

For consistency, the same reference numerals are used to denote similar elements illustrated throughout the drawings.

### DETAILED DESCRIPTION OF PREFERRED EMBODIMENTS

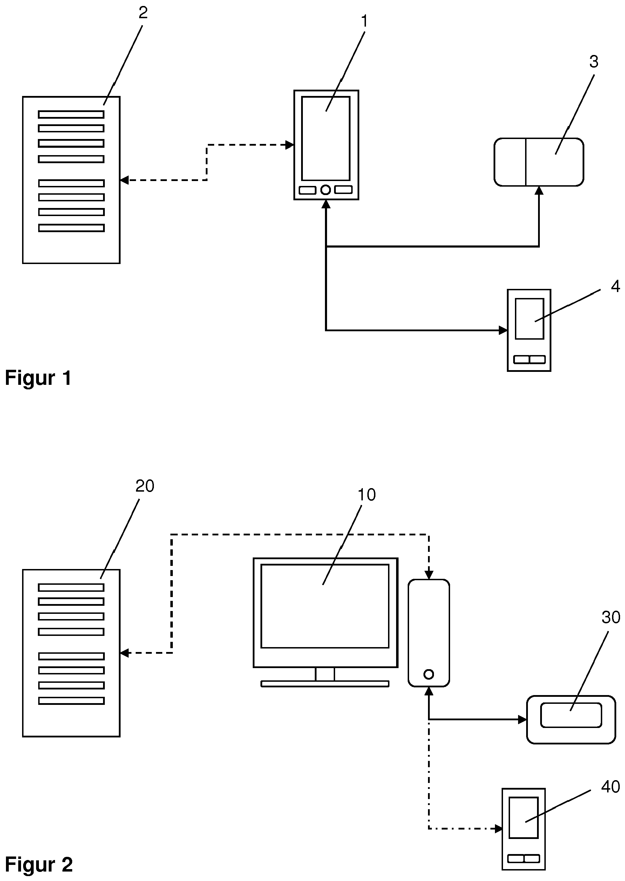

Figure 1 shows schematically an arrangement with a computing device according to the invention in the form of a mobile smart phone 1 with a computer program adapted to carry out the steps of the method according to the invention in order to prepare time related data of medical devices connected to the mobile phone 1. The arrangement comprises the mobile phone 1, a time server 2, as well as a blood glucose meter (BGM) 4 and an insulin patch pump 3 as two exemplary medical devices. In another embodiment, the arrangement may comprise a conventional insulin pump instead of the patch pump 3. The mobile phone 1 is wirelessly connected to the time server 2 via internet, whereas the patch pump 3 and the meter 4 are connected via Bluetooth Low Energy (BLE) to the mobile phone 1. The patch pump 3 can be controlled by a control software running on an external control device remote from the patch pump, which pump external device may eventually coincide with the mobile phone 1. In this case, medical device data may be provided by the patch pump itself and/or by the patch pump control software. The external time server 2 is providing the Coordinated Universal Time (UTC) as a reference time. A mobile device software application (named hereinafter as app) is running on the mobile phone 1 as a computer program.

In the following and referring to figure 1 the method of preparing of the time related data is described in detail.

The app is adapted to import data from the medical devices, the data previously recorded by the medical devices. Furthermore, the app is adapted to process and store the data. For example, the app provides a bolus calculator with an insulin on board (IOB) calculation function. Finally, the app allows to display the imported and processed data. For example, the app displays in a graph on a display of the mobile phone 1 a measured blood glucose value, amounts of delivered insulin, a computed bolus, a basal rate and the IOB.

The mobile phone 1 comprises a counter, which counts the seconds since startup of the mobile phone 1. The current counted seconds produce a so-called app system up time or computer program system up time. When the app is set up it establishes a wireless connection via internet to the UTC time server 2. After each startup of the app it computes and stores a time offset between the UTC and the app system up time. The time offset is also updated and stored at every UTC time server synchronization as described below, which is executed at least once within 24 hours.

In addition to the app system up time the app maintains an app UTC time as a synchronized time of the computer program, which is periodically synchronized with the UTC of the UTC time server 2. Furthermore, the app additionally comprises a local app time which corresponds to a time base of the mobile phone such as a display time. The local app time may be set manually by the user. The local app time is displayed by the mobile phone and may be adjusted, for example, when travelling to another time zone.

If the UTC time server 2 is not accessible e.g. if the app is not connected to the internet, the app adds the computed time offset to the current app system up time in order to compute a temporary UTC or reference time base for synchronizing the app UTC time. In this case, it has to be ensured that the mobile phone 1 has not been rebooted. Rebooting the mobile phone 1 resets the counter and thus renders the counter unusable for the computation of the temporary UTC time base.

Therefore, the app checks if the mobile phone has been rebooted since the last instant of time offset calculation, before the temporary UTC time base is computed. For this purpose, the app compares the current mobile phone system up time and the app system up time at the last app startup. A decrease in system up time may be a sufficient condition of a reboot having occurred in the meantime, in which case the app prompts the user to connect the app to the internet in order to synchronize the app UTC time.

Alternatively, if the mobile phone 1 has been rebooted and if the UTC time server 2 is not accessible either the app uses the UTC offset of the patch pump 3 to synchronize the app UTC time. The UTC offset of the patch pump 3 will be described below.

If both the mobile phone 1 and the pump have been rebooted any UTC time base is lost. In this case, the UTC time server 2 must be used to synchronize the app UTC time. The app prompts the user to connect the app to the internet in order to access the UTC time server 2. The importing of data from devices connected to the app is blocked until the app UTC time is synchronized with the UTC.

The insulin patch pump 3 contains an internal real time clock (RTC) that keeps the local time as a time base of the medical device. The RTC can be set manually by the user from the app of the mobile phone 1. If a conventional insulin pump is used instead of a patch pump 3 the user can set the local time as a display time directly on a screen of the conventional pump. In the following the RTC time is named as pump local time. Beside the pump local time, the pump comprises a counter which counts the seconds since startup of the pump and provides a pump system up time as another time base of the medical device. The counter is stored when the patch pump 3 enters in idle mode and continues when the patch pump 3 is restarted. This counter is used as a control mechanism when determining the temporary UTC time base with the app based on the UTC offset of the patch pump as described below.

The patch pump 3 records events such as a delivery of insulin to the user or a time change of the pump local time. The events are stored along with the local time and the pump system up time. Hence, the time stamp of the patch pump 3 comprises both, the local time and the pump system up time.

The pump local time may be adjusted and set to the local app time automatically if the time differences is smaller than 10 minutes. The time changes are recorded as time change event and stored in a logbook of the patch pump 3. For time changes smaller than one minute the patch pump 3 does not generate a time change event. Thus, the logbook does not comprise logbook events which are of no interest. If the time difference is greater than 10 minutes the app prompts the user to confirm the time synchronization of the pump local time with the app local time. However, data from the patch pump 3 can be imported by the app on the mobile phone 1 with or without adjustment of the pump local time.

The app computes an UTC offset between the pump system up time and the UTC and sends the computed UTC offset to the pump 3. The pump 3 stores the UTC offset. If the patch pump 3 is in idle mode and thus not able to provide a pump system up time to the app the UTC offset is set to zero. A zero offset can be interpreted by the app as an invalid offset and thus the idle mode of the pump can be recognized by the app.

In case of a time change of the pump local time the pump 3 stores an event in the logbook of the pump 3. The pump local time may be changed, for example, if the user is traveling through different time zones and therefore adjusting the local time of the pump 3 to the time zone reached. The stored data contain the time of the change (previous local time) as well as the new pump local time.

The blood glucose meter 4 (named herein as BGM) stores the blood glucose measurements as events along with a time stamp of a BGM time in its logbook. The BGM time is a time base such as a display time or local time of a medical device. The BGM time is likely to differ from the pump local time of the pump 3.

The BGM time is set to the app local time automatically if the time difference between the BGM time and the app local time greater than 2 minutes and smaller than 10 minutes. If the time difference is greater than 10 minutes the app blocks the import of the data from the BGM 4 to the app. In this case, the BGM 4 prompts the user to confirm the time synchronization with the app local time. If the BGM time is changed the BGM 4 stores a time change event. The stored data comprise the time of the change (previous BGM time) as well as the new BGM time.

The app collects data from the medical devices connected to the app by importing the events with the corresponding time stamps of the events. Relating to the arrangement shown in figure 1 the app periodically requests data from the pump 3 and from the BGM 4.

First of all the app computes a time difference between the app UTC time and the pump local time and between the app UTC time and the BGM time.

If an absolute value of the computed time difference between the app UTC time and the pump local time exceeds 24 hours the user is informed by a message displayed by the app and the importing of data is blocked by the app. A time difference greater than 24 hours cannot be caused by a time zone change and is erroneous to the extent that manual or human intervention is unavoidable in order to reconcile the pump local time and the app UTC time.

If an absolute value of the computed time difference between the app UTC time and the BGM time exceeds 10 minutes the user is informed by a message displayed by the app and the importing of data is blocked.

If the time difference is smaller than 24 hours relating to the pump 3 or smaller than 10 minutes relating to the BGM 4 respectively, the app starts to import the event data along with the time stamps stored in the logbook of the patch pump 3 and the logbook of the BGM 4 respectively. The app imports the logbook data in the sequence of the event storage. The order of the sequence is preserved in any case.

After importing the data the app converts the time stamps generated by the pump to new UTC time stamps. For that purpose, the app computes the time difference between the app UTC time and the pump system up time. As for the calculation the pump system up time is used instead of the pump local time any possible time changes of the pump local time have no impact on the calculations. The computed time difference is added to the pump system up time stamp resulting in a new UTC time stamp. The original time stamp and the new UTC time stamp are both stored by the app.

In case the time difference between the app UTC time and the pump system up time cannot be computed, e.g. due to a UTC timeline gap caused by an error or a reboot of the pump, the app converts the time stamp by adding a time difference between UTC and the phone time to the pump local time stamp (time stamp generated with the local time of the pump instead of the pump system up time).

If events are detected that have been recorded before a pump restart or before a pump error the events are marked as invalid by the app. Such invalid events are not used by the app for calculations of the insulin on board.

A similar procedure applies for the data imported from the BGM 4. The app computes a time difference between the app UTC time and the BGM time and adds the difference to the time stamp generated by the BGM 4 resulting in a new UTC time stamp.

However, any time change by the user in the BGM 4 has an impact on the time stamps generated by the BGM 4. Therefore, the time change events have to be considered when importing data from the BGM 4. If the app detects a time change event the app computes a time delta between the previous BGM time and the new BGM time. The delta time may be a positive or a negative value. Subsequently, the app adds the delta time to the time stamps generated with the previous BGM time. This is repeated for all time stamps of the current import and for all time changes of the BGM time that may affect the imported time stamps. Finally, the time difference between the app UTC time and the latest BGM time is added to the BGM time stamps. This yields to the new and additional UTC time stamp.

The app stores both, the original imported time stamp from the patch pump 3 or from the BGM 4 in its database along with the converted pump UTC time stamps and BGM UTC time stamps. All time stamps are stored along with the event data related to the time stamp.

The event data form the patch pump 3 and the BGM 4 are displayed in graphs, tables or lists generated by the app and shown on a display of the mobile phone 1. For that purpose, the converted UTC time stamps are again converted to a display time stamp by adding a time difference between the current local app time and the current app UTC time.

If the user uses two insulin pumps or switches from one pump to another only the data of the currently used pump which are newer than the data of the previously used pump are displayed. Therefore, there cannot be any overlapping of data from different pumps.

In figure 2 another embodiment of the arrangement with the computing device, the computer program and the method according to the invention is shown.

In contrast to the arrangement descripted in relation to figure 1 the arrangement shown in figure 2 comprises a personal desktop computer 10 (named hereinafter PC) instead of a mobile phone 1 and instead of an insulin patch pump 3 the PC is connected to a conventional insulin pump 30 with tubing. The PC 10 is further connected to an external UTC time server 20 and to a BGM 40. The BGM 40 is connected via USB to the PC 10 (dash-dot line in figure 2) and the pump 30 is connected wirelessly via Bluetooth Low Energy (BLE) to the PC 10. In another embodiment the BGM 40 may be connected by Bluetooth or by any other short range communication network to the computer depending on the connection possibilities of the BGM 40 and the PC 10.

A PC software program running on the PC 10 is adapted to organize the preparing of the time related data according to the invention. The function of the program mainly corresponds to the function of the app of the mobile phone 1 described above in relation to figure 1.

In contrast to the above descripted arrangement the PC system up time is not used. Instead a PC UTC time is used for converting the time stamps generated by the pump 30 and the BGM 40. The PC UTC time is periodically synchronized with the UTC time server. Consequently, the PC software program computes a time difference between the PC UTC time and the pump local time and adds the difference to the time stamp generated by the pump local time.

The invention is not limited to the arrangement above. For example, the app may be connected to a continuous glucose monitoring (CGM) device instead of a BGM. Furthermore, the PC software may be connected to a patch pump instead of an insulin pump with tubing. Additionally or instead the PC may be connected to an electronic module attached to an injection pen and adapted to record a dose setting, an amount of the injection and parameter settings of the injection pen. The electronic module records and stores the data along with a time stamp. The app or the PC software may convert the time stamp from the electronic module in the same way as the time stamp from the BGM.

While the invention has been described in detail in the drawings and foregoing description, such description is to be considered illustrative or exemplary and not restrictive.

### LIST OF REFERENCE NUMERALS

- 1: mobile phone
- 2, 20: UTC time server
- 3: insulin patch pump
- 4, 40: blood glucose meter (BGM)
- 10: personal desktop computer (PC)
- 30: insulin pump with tubing

## Claims

1. A method for preparing, by a computing device (1), time related data from a medical device (3, 4) connected to the computing device, comprising the steps
a. computing a time difference between a synchronized time of a computer program executed on the computing device and a time of the medical device, wherein the synchronized time of the computer program is synchronized with a reference time;
b. comparing the time difference with a predefined condition;
c. importing, or not importing, data with a time stamp from the medical device depending on the comparison; and
d. generating, if imported, a converted time stamp from the time stamp of the imported data and based on the synchronized time of the computer program.

2. Method according to claim 1, **characterized in that** the reference time is the Coordinated Universal Time UTC.

3. Method according to claim 1 or 2, **characterized in that** the data are not imported if an absolute value of the time difference exceeds a first threshold, wherein the first threshold preferably equals 24 hours.

4. Method according to any of claims 1 to 3, **characterized in that** the condition includes a second threshold and the user is informed if the time difference exceeds the second threshold.

5. Method according to any of claims 1 to 4, **characterized in that** the generation of the converted time stamp comprises the step of adding the time difference to the time stamp of the imported data.

6. Method according to any of claims 1 to 5, **characterized in that** the reference time is computed by adding a time offset to a computer program system up time, wherein the time offset is a previously computed difference between the computer program system up time and one of the synchronized time of the computer program and the reference time.

7. Method according to claim 6, **characterized by** the step of verifying that the computer program system up time has not been reset.

8. Method according to claim 6 or 7, **characterized in that** the reference time is computed by adding a time offset to a time base of the medical device, wherein the time offset is a previously computed difference between the time base of the medical device and the reference time.

9. Method according to any of claims 1 to 8, **characterized in that** the data are related to an event, preferably to a measurement of a bodily fluid, a delivery of a drug, or a time change of a time of the medical device.

10. Method according to claim 9, **characterized in that** if an event relating to a time change of a time base of the medical device is identified by the computing device, a delta time corresponding to the change is added to the time stamps generated prior to the time change.

11. Method according to any of claims 1 to 10, **characterized in that** the imported time stamp is marked or the imported data related to the time stamp are marked if the time stamp is not within a predefined expected range.

12. Method according to any of claims 1 to 11, **characterized in that** the medical device is a blood glucose measuring device (4) or an infusion pump (3).

13. Computer program product comprising instructions which, when executed by a computing device, cause the computing device to carry out the method according to any of claim 1 to 12.

14. A computing device comprising processing means configured for carrying out the steps of the method according to any of claim 1 to 12.

15. A computing device according to claim 14, **characterized in that** the computing device is a mobile device.
